# EUROPEAN PATENT APPLICATION

(11) **EP 1 550 455 A1**
(43) Date of publication of application: **06.07.2005**
(21) Application number: 03730609.9
(22) Date of filing: 23.05.2003
(51) Int. Cl.: A61K 38/43, A61K 39/395, A61K 45/00, A61P 7/02, A61P 7/04

(54) **AGENT NEUTRALIZINT TISSUE FACTOR INHIBITOR AND AGENT NEUTRALIZING ACTIVATED BLOOD COAGULATION FACTOR VIII PREPARATION**

(30) Priority: 23.05.2002 JP 2002149494
(71) Applicant: CHUGAI SEIYAKU KABUSHIKI KAISHA, Tokyo, 115-8543 (JP)
(72) Inventor: KITAZAWA, Takehisa c/o Chugai Seiyaku K. K., Gotenba-shi, Shizuoka 412-8513 (JP); HATTORI, Kunihiro c/o Chugai Seiyaku K. K., Gotenba-shi, Shizuoka 412-8513 (JP)
(74) Representative: Holliday, Louise Caroline
(86) International application number: PCT/JP2003/006469
(87) International publication number: WO 2003/099324

(57) **Abstract**

Formulations for neutralizing the anticoagulant action of a human tissue factor inhibitor, comprising a blood coagulation factor complex product or an activated blood coagulation factor VII product as an active ingredient; and formulations for neutralizing the blood coagulation-promoting action of an activated blood coagulation factor VII product, comprising a human tissue factor inhibitor as an active ingredient.

Sequence Listing

<110> Chugai Seiyaku Kabushiki Kaisha

<120> Neutralizers of Tissue Factor Inhibitors and Neutralizers of Activated

Blood Coagulation Factor VII Products

<130> YCT-808

<160> 2

<210> 1

<211> 444

<212> PRT

<213>

<400> 1

Gln Val Gln Leu Leu Glu Ser Gly Ala Val Leu Ala Arg Pro Gly Thr

                5                   10                  15

Ser Val Lys Ile Ser Cys Lys Ala Ser Gly Phe Asn Ile Lys Asp Tyr

            20                  25                  30

Tyr Met His Trp Val Lys Gln Arg Pro Gly Gln Gly Leu Glu Trp Ile

        35                  40                  45

Gly Gly Asn Asp Pro Ala Asn Gly His Ser Met Tyr Asp Pro Lys Phe

    50                  55                  60

Gln Gly Arg Val Thr Ile Thr Ala Asp Thr Ser Thr Ser Thr Val Phe

65                  70                  75                  80

Met Glu Leu Ser Ser Leu Arg Ser Glu Asp Thr Ala Val Tyr Tyr Cys

                85                  90                  95

Ala Arg Asp Ser Gly Tyr Ala Met Asp Tyr Trp Gly Gln Gly Thr Leu

            100                 105                 110

Val Thr Val Ser Ser Ala Ser Thr Lys Gly Pro Ser Val Phe Pro Leu

115                 120                 125

Ala Pro Cys Ser Arg Ser Thr Ser Glu Ser Thr Ala Ala Leu Gly Cys

    130                 135                 140

Leu Val Lys Asp Tyr Phe Pro Glu Pro Val Thr Val Ser Trp Asn Ser

145                 150                 155                 160

Gly Ala Leu Thr Ser Gly Val His Thr Phe Pro Ala Val Leu Gln Ser

                165                 170                 175

Ser Gly Leu Tyr Ser Leu Ser Ser Val Val Thr Val Pro Ser Ser Ser

            180                 185                 190

Leu Gly Thr Lys Thr Tyr Thr Cys Asn Val Asp His Lys Pro Ser Asn

        195                 200                 205

Thr Lys Val Asp Lys Arg Val Glu Ser Lys Tyr Gly Pro Pro Cys Pro

    210                 215                 220

Pro Cys Pro Ala Pro Glu Phe Leu Gly Gly Pro Ser Val Phe Leu Phe

225                 230                 235                 240

Pro Pro Lys Pro Lys Asp Thr Leu Met Ile Ser Arg Thr Pro Glu Val

                245                 250                 255

Thr Cys Val Val Val Asp Val Ser Gln Glu Asp Pro Glu Val Gln Phe

            260                 265                 270

Asn Trp Tyr Val Asp Gly Val Glu Val His Asn Ala Lys Thr Lys Pro

        275                 280                 285

Arg Glu Glu Gln Phe Asn Ser Thr Tyr Arg Val Val Ser Val Leu Thr

    290                 295                 300

Val Leu His Gln Asp Trp Leu Asn Gly Lys Glu Tyr Lys Cys Lys Val

305                 310                 315                 320

Ser Asn Lys Gly Leu Pro Ser Ser Ile Glu Lys Thr Ile Ser Lys Ala

                325                 330                 335

Lys Gly Gln Pro Arg Glu Pro Gln Val Tyr Thr Leu Pro Pro Ser Gln

            340                 345                 350

Glu Glu Met Thr Lys Asn Gln Val Ser Leu Thr Cys Leu Val Lys Gly
        355                 360                 365
Phe Tyr Pro Ser Asp Ile Ala Val Glu Trp Glu Ser Asn Gly Gln Pro
    370                 375                 380
Glu Asn Asn Tyr Lys Thr Thr Pro Pro Val Leu Asp Ser Asp Gly Ser
385                 390                 395                 400
Phe Phe Leu Tyr Ser Arg Leu Thr Val Asp Lys Ser Arg Trp Gln Glu
                405                 410                 415
Gly Asn Val Phe Ser Cys Ser Val Met His Glu Ala Leu His Asn His
            420                 425                 430
Tyr Thr Gln Lys Ser Leu Ser Leu Ser Leu Gly Lys
        435                 440

<210> 2
<211> 214
<212> PRT
<213>
<400> 2

Asp Ile Gln Met Thr Gln Ser Pro Ser Ser Leu Ser Ala Ser Val Gly
                5                   10                  15
Asp Arg Val Thr Ile Thr Cys Lys Ala Ser Gln Asp Ile Lys Ser Phe
            20                  25                  30
Leu Ser Trp Tyr Gln Gln Lys Pro Glu Lys Ala Pro Lys Ser Leu Ile
        35                  40                  45
Tyr Tyr Ala Thr Ser Leu Ala Asp Gly Val Pro Ser Arg Phe Ser Gly
    50                  55                  60
Ser Gly Ser Gly Thr Asp Tyr Thr Leu Thr Ile Ser Ser Leu Gln Pro
65                  70                  75                  80

Glu Asp Phe Ala Thr Tyr Tyr Cys Leu Gln His Gly Glu Ser Pro Tyr
                85                  90                  95
Thr Phe Gly Gly Gly Thr Lys Val Glu Ile Lys Arg Thr Val Ala Ala
            100                 105                 110
Pro Ser Val Phe Ile Phe Pro Pro Ser Asp Glu Gln Leu Lys Ser Gly
        115                 120                 125
Thr Ala Ser Val Val Cys Leu Leu Asn Asn Phe Tyr Pro Arg Glu Ala
    130                 135                 140
Lys Val Gln Trp Lys Val Asp Asn Ala Leu Gln Ser Gly Asn Ser Gln
145                 150                 155                 160
Glu Ser Val Thr Glu Gln Asp Ser Lys Asp Ser Thr Tyr Ser Leu Ser
                165                 170                 175
Ser Thr Leu Thr Leu Ser Lys Ala Asp Tyr Glu Lys His Lys Val Tyr
            180                 185                 190
Ala Cys Glu Val Thr His Gln Gly Leu Ser Ser Pro Val Thr Lys Ser
        195                 200                 205
Phe Asn Arg Gly Glu Cys
    210

## Description

### TECHNICAL FIELD

The present invention relates to neutralizers of the anticoagulant action of human tissue factor inhibitors, comprising a blood coagulation factor complex product or an activated blood coagulation factor VII product as an active ingredient. The present invention also relates to neutralizers of the blood coagulation-promoting action of activated blood coagulation factor VII products, comprising a human tissue factor inhibitor as an active ingredient.

### BACKGROUND ART

Blood coagulable reaction is a cascade of reactions in which the activated serine proteases activate the next serine proteases one after another to finally form fibrin. Thrombosis arises as a result of the blood coagulable reaction triggered and excessively promoted by the functional changes of the coagulation/fibrinolytic systems, platelets, leucocytes in plasma and/or vascular endothelial cells accompanying the progress of various pathologic conditions.

Tissue factor (TF) is a cell-surface receptor of blood coagulation factor VII and has been positioned as a substantial trigger of the blood coagulable reaction. TF activates blood coagulation factors IX and X by forming a complex with blood coagulation factor VII. TF normally scarcely exists in tissues exposed to the bloodstream such as vascular endothelium or blood cells. However, TF abundantly exists in vascular adventitia and connective tissues. Such TF is thought to activate the blood coagulation system to serve a hemostatic function when blood vessel is damaged.

The syndromes of disseminated intravascular coagulation (DIC) are diseases in which numerous small blood clots develop mainly in small blood vessels throughout the body by the activation of the blood coagulation system in blood vessels. It can be regarded as an acquired excessive hypercoagulable state. Multiple small blood clots induce microcirculation failure in major organs, occasionally leading to irreversible dysfunction in the organs. Bleeding also often occurs due to the consumption and loss of platelets or blood coagulation factors, although it is a reverse phenomenon to clotting. Therefore, DIC is recognized as a serious disease because it is often associated with poor prognosis. DIC occurs as a complication in various diseases such as acute leukemia, malignant lymphoma and infectious diseases, but results from a common causal agent TF. Thus, DIC is attributed to the following etiological mechanisms: abnormally increased TF production/expression of tumor cells in acute leukemia, malignant lymphoma and solid cancer; increased TF production/expression in monocytes/vascular endothelial cells in infectious diseases (especially, gram-negative septicemia); influx of TF from necrotic liver tissue into the bloodstream in fulminant hepatitis; TF formation on the inner surfaces of vessels in aortic aneurysm, cardiac aneurysm and huge hemangioma; influx of TF in placental tissue into the bloodstream in obstetrical diseases (amniotic fluid embolism, premature separation of a normally implanted placenta); and influx of TF in injured tissue into the bloodstream in surgery, trauma and burn.

Current therapies for DIC include anticoagulation therapy using heparin products or the like and replacement therapy using concentrated platelet plasma or the like. Recently, a new approach to DIC therapy has been studied by neutralizing the activity of TF using anti-TF antibodies or the like. Anti-TF antibodies are expected to serve as safe and effective anticoagulants with few adverse side effects such as bleeding tendency. Especially, humanized anti-TF antibodies are expected to be excellent therapeutic agents, which are safe and effective and remain in blood for a long period.

Arterial thrombosis is a disease in which clots are formed in blood vessels with advanced sclerosis, and which is often fatal when it develops in a major organ such as brain and heart. Especially, coronary syndromes such as unstable angina or acute myocardial infarction are considered to be a dangerous pathological condition leading to sudden death. Recently, it has become clear that atherosclerotic plaque rupture and the subsequent clot formation are major factors in the etiological mechanism. It has also been shown that TF, a trigger of clot formation, is overexpressed on cell surfaces and in extracellular stroma in plaques, suggesting that the exposure of TF to the bloodstream subsequent to plaque rupture is a major factor in clot formation.

In venous thrombosis, congestion of venous bloodstream, damage of venous walls and enhancement of blood coagulability are regarded as the critical factors in the etiological mechanism. Especially, invasions such as surgery, delivery and trauma invite physical damages to blood walls and abnormality in the coagulation/fibrinolytic system, and postoperative bed rest congests venous bloodstream. The resulting venous blood clots cause not only circulation failure through limbs but also fatal pulmonary embolism via influx of clots per se along the bloodstream into the pulmonary artery, which means that the prevention of venous thrombosis per se is important.

We prepared human/mouse chimeric antibodies consisting of the variable regions (V regions) of a mouse monoclonal antibody against human TF and the constant regions (C regions) of a human antibody, as well as humanized antibodies consisting of the complementarity-determining regions in the light chain (L chain) variable region and the heavy chain (H chain) variable region of a mouse monoclonal antibody against human TF transferred into a human antibody, and reported that these could be expected as excellent therapeutic agents for DIC, arterial thrombosis and venous thrombosis (WO99/51743, WO01/24626).

However, unexpected accidents may occur during the use of anti-TF antibodies to treat various diseases as mentioned above in the actual clinical field, though they are anticoagulants with less bleeding tendency. Thus, the presence of a convenient and rapid means for neutralizing the activity of anti-TF antibodies in case of bleeding tendency or other events induced by the anti-TF antibodies is important in terms of drug safety.

Activated blood coagulation factor VII products are marketed as anti-bleeding agents for hemophiliac patients with inhibitors. Hemophiliac patients hereditarily lack the ability of activating blood coagulation factor X by the activated blood coagulation factor VIII / activated blood coagulation factor IX complex. When hemophiliac patients bleed, they are normally treated by administration of blood coagulation factor VIII products (for hemophilia A) or blood coagulation factor IX products (for hemophilia B). However, blood coagulation factors contained in these products are "foreign substances" for patients with severe hemophilia, whereby inhibitors (antibodies) may be produced. In this case, the effect of these products will be considerably reduced.

Activated blood coagulation factor VII products can induce a hemostatic action even in hemophiliac patients with inhibitors, because they can activate blood coagulation factor X in the presence of TF. However, some reports showed the appearance of thrombosis, probably due to the blood coagulation-promoting action of activated blood coagulation factor VII products.

Recently, activated blood coagulation factor VII products have become recognized to be effective for not only hemophilic patients but also for common severe bleeding such as subarachnoid hemorrhage, intracerebral hemorrhage, aneurysm rupture and trauma. For example, several reports come from the clinical field, which demonstrate that activated blood coagulation factor VII products showed a very dramatic hemostatic effect for patients with massive bleeding after injury in traffic accidents. Massive bleeding is an emergency condition, for which the effectiveness of activated blood coagulation factor VII products seems indisputable. However, hypercoagulable states are also often induced by infectious diseases or influx of damaged tissue into the bloodstream or other causes in such patients. Hypercoagulable states may lead to severe conditions such as DIC or thrombosis. In this case, activated blood coagulation factor VII products act to aggravate such conditions.

Thus, it would be clinically very useful to rapidly neutralize the blood coagulation-promoting action of activated blood coagulation factor VII products without rebleeding after hemostasis has been achieved by the activated blood coagulation factor VII products.

An object of the present invention is to provide a prophylactic or therapeutic agent capable of neutralizing the activity of tissue factor inhibitors such as anti-TF antibodies in cases where the tissue factor inhibitors have induced or may induce bleeding tendency. Another object of the present invention is to provide a prophylactic or therapeutic agent capable of neutralizing the activity of activated blood coagulation factor VII products in cases where the activated blood coagulation factor VII products have induced or may induce thrombosis or hypercoagulable states.

### DISCLOSURE OF THE INVENTION

As a result of careful studies to develop a prophylactic or therapeutic agent capable of neutralizing the activity of anti-TF antibodies in cases where the anti-TF antibodies have induced or may induce bleeding tendency, we accomplished the present invention on the basis of the finding that the anticoagulant action of the anti-TF antibodies can be neutralized by administering a blood coagulation factor complex product or an activated blood coagulation factor VII product.

As a result of careful studies to develop a prophylactic or therapeutic agent capable of neutralizing the activity of activated blood coagulation factor VII products in cases where they have induced or may induce thrombosis or hypercoagulable states, we also accomplished the present invention on the basis of the finding that the blood coagulation-promoting action of the activated blood coagulation factor VII products can be neutralized by administering a human TF inhibitor.

Accordingly, the present invention provides formulations for neutralizing the anticoagulant action of a human TF inhibitor, comprising a blood coagulation factor complex product or an activated blood coagulation factor VII product as an active ingredient.

The present invention also provides formulations for suppressing the bleeding or bleeding tendency caused by a human TF inhibitor, comprising a blood coagulation factor complex product or an activated blood coagulation factor VII product as an active ingredient.

The present invention also provides formulations for neutralizing the blood coagulation time (prothrombin time) prolonging action caused by a human TF inhibitor, comprising a blood coagulation factor complex product or an activated blood coagulation factor VII product as an active ingredient.

The present invention also provides formulations for suppressing bleeding or bleeding tendency, comprising a blood coagulation factor complex product or an activated blood coagulation factor VII product as an active ingredient, characterized in that it is administered simultaneously with a human TF inhibitor or after administering a human TF inhibitor.

The present invention also provides antithrombotic agents comprising a human TF inhibitor and a blood coagulation factor complex product or an activated blood coagulation factor VII product.

The present invention also provides pharmaceutical kits comprising a human TF inhibitor and a blood coagulation factor complex product or an activated blood coagulation factor VII product.

The present invention also provides formulations for suppressing the blood coagulation-promoting action of an activated blood coagulation factor VII product, comprising a human TF inhibitor as an active ingredient.

The present invention also provides formulations for suppressing the thrombosis or hypercoagulable states caused by an activated blood coagulation factor VII product, comprising a human TF inhibitor as an active ingredient.

The present invention also provides formulations for neutralizing the blood coagulation time (prothrombin time) shortening action caused by an activated blood coagulation factor VII product, comprising a human TF inhibitor as an active ingredient.

The present invention also provides formulations for suppressing thrombosis or hypercoagulable states, comprising a human TF inhibitor as an active ingredient, characterized in that it is administered simultaneously with an activated blood coagulation factor VII product or after administering an activated blood coagulation factor VII product.

The present invention also provides hemostatic agents comprising an activated blood coagulation factor VII product and a human TF inhibitor.

In these neutralizers, suppressants, antithrombotic agents and pharmaceutical kits, the human TF inhibitor is preferably an anti-human TF antibody, more preferably a humanized anti-human TF antibody. Most preferably, the humanized anti-human TF antibody is a humanized anti-human TF antibody comprising the humanized H chain version shown in SEQ ID NO: 1 and the humanized L chain version shown in SEQ ID NO: 2.

### BRIEF DESCRIPTION OF THE DRAWINGS

Figure 1 shows the effect of Proplex ST on the PT-prolonging action induced by a humanized anti-human TF antibody.
Figure 2 shows the effect of PPSB-HT on the PT-prolonging action induced by a humanized anti-human TF antibody.
Figure 3 shows the effect of Autoplex on the PT-prolonging action induced by a humanized anti-human TF antibody.
Figure 4 shows the effect of Novact M on the PT-prolonging action induced by a humanized anti-human TF antibody.
Figure 5 shows the effect of Recombinate on the PT-prolonging action induced by a humanized anti-human TF antibody.
Figure 6 shows the effect of a Novact M/Recombinate combination on the PT-prolonging action induced by a humanized anti-human TF antibody.
Figure 7 shows the effect of Albuminate on the PT-prolonging action induced by a humanized anti-human TF antibody.
Figure 8 shows the effect of an activated blood coagulation factor VII product on the PT-prolonging action induced by a humanized anti-human TF antibody.
Figure 9 shows the effect of a humanized anti-human TF antibody on the PT-shortening action induced by an activated blood coagulation factor VII product.

### THE MOST PREFERRED EMBODIMENTS OF THE INVENTION

As used herein, human TF inhibitors are not specifically limited in so far as they act on human TF to show an anticoagulant action, but are preferably anti-human TF antibodies. Human TF recognized by these anti-human TF antibodies may be simple human TF or complexes with physiologically active factors. Examples of physiologically active factors are preferably those binding to human TF to induce blood coagulation states. Specific examples of said physiologically active factors include blood coagulation factors, more preferably, blood coagulation factor VII and blood coagulation factor X, which may be activated or not.

The anti-human TF antibodies may be monoclonal or polyclonal antibodies, but preferably monoclonal antibodies because homogeneous antibodies can be stably produced.

The anti-human TF antibodies may be mouse antibodies, human antibodies, chimeric antibodies, humanized antibodies and the like as appropriate, but preferably humanized anti-human TF antibodies, especially humanized anti-human TF antibodies comprising a combination of the humanized H chain version shown in SEQ ID NO: 1 and the humanized L chain version shown in SEQ ID NO: 2 (humanized H chain version i and humanized L chain version b2 described in WO99/51743).

Many anti-human TF antibodies have already been reported (WO99/51743, WO88/07543, WO96/40921, WO98/40408, WO01/70984, WO03/037911). They can be prepared by methods known to those skilled in the art because their antigen TF is already known (Ito T et al., J. Biochem. 114, 691-696, (1993)).

Active ingredients in the formulations for neutralizing the anticoagulant action of a human TF inhibitor or formulations for suppressing the bleeding or bleeding tendency caused by a human TF inhibitor according to the present invention are blood coagulation factor complex products or activated blood coagulation factor VII products.

As used herein, blood coagulation factor complex products include blood coagulation factor complex concentrate products (common name given in the Japanese Pharmacopoeia: freeze-dried human blood coagulation factor IX complexes), activated blood coagulation factor complex concentrate products (common name given in the Japanese Pharmacopoeia: freeze-dried human blood coagulation factor antibody bypassing activity complexes, activated prothrombin complexes), etc. Blood coagulation factor complex products currently on the market include, for example, the following:
1) Blood coagulation factor complex concentrate products (common name given in the Japanese Pharmacopoeia: freeze-dried human blood coagulation factor IX complexes) are commercially available in Japan as PPSB-HT "Nichiyaku"®(Nihon Pharmaceutical - Takeda Pharmaceutical), Proplex ST® (Baxter), and BENOBIL TIM4® (Baxter); and commercially available abroad as Konyne 80® (Bayer), Proplex T® (Baxter - Hyland), Profilnine HT® (Alpha therapeutic), and Bebulin® (Immuno);
2) Activated blood coagulation factor complex concentrate products (common name given in the Japanese Pharmacopoeia: freeze-dried human blood coagulation factor antibody bypassing activity complexes, activated prothrombin complexes) are commercially available in Japan as FEIBA ® (Baxter), FEIBA "Immuno"® (Nippon Zoki Pharmaceutical), and Autoplex® (Baxter); and commercially available abroad as Autoplex® (Baxter - Hyland), and FEIBA VH Immuno® (Immuno).

Activated blood coagulation factor VII products (recombinant activated blood coagulation factor VII products) are commercially available in Japan as NovoSeven ® (Novo Nordisk); and commercially available abroad as NoveSeven® or NiaState® (Novo Nordisk). As used herein, activated blood coagulation factor VII products may also contain other components so far as they contain an activated blood coagulation factor VII product.

Blood coagulation factor VII products are more advantageously activated. Only activated blood coagulation factor VII products are currently commercially available, but non-activated blood coagulation factor VII products are also included in the present invention in so far as they have the suppressive or neutralizing action according to the present invention.

One or more of these blood coagulation factor complex products or activated blood coagulation factor VII products can be used alone or in combination.

Whether a blood coagulation factor complex product or an activated blood coagulation factor VII product neutralizes the anticoagulant action of a human TF inhibitor or whether it suppresses the bleeding or bleeding tendency caused by a human TF inhibitor can be determined on the basis of the prothrombin time (sometimes hereinafter also referred to as PT). Similarly, whether a human TF inhibitor shows an anticoagulant action can also be determined on the basis of the prothrombin time. Moreover, whether a human TF inhibitor suppresses the blood coagulation-promoting action of an activated blood coagulation factor VII product, or whether it suppresses the thrombosis or hypercoagulable states caused by an activated blood coagulation factor VII product, or whether it neutralizes the blood coagulation time (prothrombin time) shortening action caused by an activated blood coagulation factor VII product can also be determined on the basis of the prothrombin time. The prothrombin time is a comprehensive kinetic assessment of the extrinsic coagulation pathway, which measures the time required for fibrin to be deposited after the addition of tissue thromboplastin and calcium chloride to a citrate plasma sample to be tested. Specifically, it can be measured by in vitro experiments using human plasma as described in the Examples below. Those products neutralizing the prothrombin time prolonging action induced by the administration of a human TF inhibitor (i.e. shortening the prothrombin time) are suitable as formulations for neutralizing the anticoagulant action of the human TF inhibitor or formulations for suppressing the bleeding or bleeding tendency caused by the human TF inhibitor according to the present invention. On the other hand, human TF inhibitors suppressing the blood coagulation-promoting action of an activated blood coagulation factor VII product (i.e. prolonging the prothrombin time) are suitable as formulations for suppressing the blood coagulation-promoting action of the activated blood coagulation factor VII product or formulations for neutralizing the blood coagulation time (prothrombin time) shortening action caused by the activated blood coagulation factor VII product according to the present invention.

In the present invention, various commercially available blood coagulation factor complex products were tested to show that the PT-prolonging action induced by a humanized anti-human TF antibody disappeared and PT decreased to levels below the normal range (11-13 seconds) in the presence of 1.88 U/mL (equivalent to 100 U/kg administered i.v.) of a therapeutic agent for hemophilia B inhibitors and hemophilia A inhibitors (Proplex ST®), a therapeutic agent for hemophilia B inhibitors (PPSB-HT®), or a therapeutic agent for hemophilia A inhibitors (Autoplex®). These three drugs are blood coagulation factor complex products, and their package leaflets state that Proplex ST contains prothrombin (factor II), factor VII, factor IX and factor X and that Autoplex contains prothrombin, factor VII, factor IX, factor X as well as activated factors VII, IX and X (no information is given about ingredients of PPSB-HT in the package leaflet). The fact that these blood coagulation factor complex products were found to neutralize the PT-prolonging action induced by a humanized anti-human TF antibody suggests that the use of blood coagulation factor complex products Proplex ST, PPSB-HT and Autoplex is effective for improving bleeding tendency if it is observed by the administration of the humanized anti-human TF antibody.

The effects of products containing a single blood coagulation factor such as Novact M® (blood coagulation factor IX product) and Recombinate® (blood coagulation factor VIII product) and a combination of these two products on the PT-prolonging action induced by the humanized anti-human TF antibody were also evaluated in the same manner.

In the presence of 1.88 U/mL (equivalent to 100 U/kg administered i.v.) of Novact M or Recombinate, the PT-prolonging action induced by the humanized anti-human TF antibody was not suppressed. When Novact M and Recombinate were used in combination, the PT-prolonging action induced by the humanized anti-human TF antibody was not suppressed. In the presence of these two products alone or in combination, the PT-prolonging action induced by the humanized anti-human TF antibody was found to rather significantly increase. In addition, PT was also significantly prolonged when these two products were used in combination even in the absence of the humanized anti-human TF antibody. These results suggest that at least blood coagulation factors VIII and IX play a minor role when a blood product neutralizes the PT-prolonging action induced by a humanized anti-human TF antibody.

The effect of activated blood coagulation factor VII on the PT-prolonging action induced by the humanized anti-human TF antibody was also evaluated in the same manner. The PT-prolonging action induced by the humanized anti-human TF antibody was neutralized in the presence of an activated blood coagulation factor VII product NovoSeven ®.

Blood coagulation factor VII is activated by forming a complex with TF, which is a blood coagulation factor VII receptor expressed on cell surfaces, to accelerate the blood coagulable reaction. Blood coagulation factor VII seems to be effective for neutralizing the PT-prolonging action induced by humanized anti-human TF antibodies because blood coagulation factor VII is also contained in blood coagulation factor complex products having the activity of neutralizing the PT-prolonging action induced by humanized anti-human TF antibodies such as Proplex ST and Autoplex.

It can be concluded from these findings that the use of activated blood coagulation factor VII and blood coagulation factor complex products containing it is effective for improving bleeding tendency if it is observed by the administration of the humanized anti-human TF antibody.

The effect of the humanized anti-human TF antibody on the PT-shortening action induced by activated blood coagulation factor VII was also evaluated. In the absence of the humanized anti-human TF antibody, an activated blood coagulation factor VII product NovoSeven ® shortened the PT time, but the PT time shortening action induced by activated blood coagulation factor VII was neutralized when the humanized anti-human TF antibody was added. Thus, human TF inhibitors such as humanized anti-human TF antibodies are effective for neutralizing the blood coagulation action of activated blood coagulation factor VII.

These findings suggest that the use of human TF inhibitors such as humanized anti-human TF antibodies is effective for improving hypercoagulable states or thrombosis induced by activated blood coagulation factor VII if it is observed.

The formulations for neutralizing the anticoagulant action of a human TF inhibitor or formulations for suppressing the bleeding or bleeding tendency caused by a human TF inhibitor comprising a blood coagulation factor complex product or an activated blood coagulation factor VII product according to the present invention can be administered in, but is not specifically limited to, cases where the human TF inhibitor has a strong anticoagulant action or the bleeding tendency caused by the human TF inhibitor is observed. They can also be administered simultaneously or sequentially or at time intervals in advance for prophylactic purposes if these situations are predicted upon administration of a human TF inhibitor. As used herein, bleeding tendency refers to a condition where potential bleeding is suspected or a condition clearly susceptible to bleeding (for example, lowered hemoglobin levels) though bleeding is not visibly observed.

The formulations for suppressing the blood coagulation-promoting action of an activated blood coagulation factor VII product or formulations for neutralizing the blood coagulation time (prothrombin time) shortening action caused by an activated blood coagulation factor VII product comprising a human TF inhibitor according to the present invention can be administered in, but are not specifically limited to, cases where the activated blood coagulation factor VII product has a strong blood coagulation-promoting action. They can also be administered simultaneously or sequentially or at time intervals in advance for prophylactic purposes if these situations are predicted upon administration of an activated blood coagulation factor VII product.

According to the present invention, the formulations can also be prepared in the form of antithrombotic agents comprising a human TF inhibitor and a blood coagulation factor complex product or an activated blood coagulation factor VII product.

According to the present invention, the formulations can also be prepared in the form of pharmaceutical kits comprising a human TF inhibitor and a blood coagulation factor complex product or an activated blood coagulation factor VII product.

According to the present invention, the formulations can also be prepared in the form of hemostatic agents comprising a human TF inhibitor and an activated blood coagulation factor VII product.

According to the present invention, the formulations can also be prepared in the form of pharmaceutical kits comprising a human TF inhibitor and an activated blood coagulation factor VII product.

The formulations or pharmaceutical kits comprising a blood coagulation factor VII product and a human TF inhibitor can function as antithrombotic agents or hemostatic agents depending on the condition of the patient (bleeding, bleeding tendency, thrombosis, hypercoagulable states, etc.), the ratio between the blood coagulation factor VII product and the human TF inhibitor, the timing of administration, etc. Whether the formulations or pharmaceutical kits comprising a blood coagulation factor VII product and a human TF inhibitor according to the present invention are to be used as antithrombotic agents or hemostatic agents can be selected as appropriate by those skilled in the art considering the condition of the patient, the ratio of the components, the timing of administration, etc.

The neutralizers, suppressants, antithrombotic agents or hemostatic agents according to the present invention are normally administered via parenteral routes, for example by injection (e.g. subcutaneous, intravenous, intramuscular or intraperitoneal injection) or via percutaneous, mucosal, nasal or pulmonary routes.

The amount of the blood coagulation factor complex product or activated blood coagulation factor VII product contained in the formulations for neutralizing the anticoagulant action of a human TF inhibitor or formulations for suppressing the bleeding or bleeding tendency caused by a human TF inhibitor or antithrombotic agents according to the present invention can be determined depending on the type of the disease to be treated, the severity of the disease, the age of the patient and other factors, but generally ranges from 0.0001 to 1000 U/kg body weight, preferably 0.01 to 10 U/kg body weight expressed as a total dose of blood coagulation factors.

The formulations for neutralizing the anticoagulant action of a human TF inhibitor or formulations for suppressing the bleeding or bleeding tendency caused by a human TF inhibitor according to the present invention can function as therapeutic agents capable of neutralizing the activity of a TF inhibitor such as an anti-human TF antibody or function as suppressants of bleeding or bleeding tendency if the TF inhibitor shows a bleeding tendency.

In the formulations for suppressing the blood coagulation-promoting action of an activated blood coagulation factor VII product comprising a human TF inhibitor as an active ingredient according to the present invention, the human TF inhibitor used is preferably an anti-human TF antibody, more preferably a humanized anti-human TF antibody. A preferred humanized anti-human TF antibody is a humanized anti-human TF antibody comprising the humanized H chain version shown in SEQ ID NO: 1 and the humanized L chain version shown in SEQ ID NO: 2.

In the formulations for suppressing the thrombosis or hypercoagulable states caused by an activated blood coagulation factor VII product or formulations for neutralizing the blood coagulation time (prothrombin time) shortening action caused by an activated blood coagulation factor VII product comprising a human TF inhibitor as an active ingredient, or formulations for suppressing thrombosis or hypercoagulable states comprising a human TF inhibitor as an active ingredient, characterized in that it is administered simultaneously with an activated blood coagulation factor VII product or after administering an activated blood coagulation factor VII product according to the present invention, the human TF inhibitor used is preferably an anti-human TF antibody, more preferably a humanized anti-human TF antibody. A preferred humanized anti-human TF antibody is a humanized anti-human TF antibody comprising the humanized H chain version shown in SEQ ID NO: 1 and the humanized L chain version shown in SEQ ID NO: 2.

In the hemostatic agents comprising an activated blood coagulation factor VII product and a human TF inhibitor according to the present invention, the human TF inhibitor used is preferably an anti-human TF antibody, more preferably a humanized anti-human TF antibody. A preferred humanized anti-human TF antibody is a humanized anti-human TF antibody comprising the humanized H chain version shown in SEQ ID NO: 1 and the humanized L chain version shown in SEQ ID NO: 2.

The amount of the human TF inhibitor contained in the formulations for suppressing the blood coagulation-promoting action or formulations for suppressing thrombosis or hypercoagulable states or formulations for neutralizing the blood coagulation time (prothrombin time) shortening action or hemostatic agents according to the present invention can be determined depending on the type of the human TF inhibitor, the type of the patient to be treated, the severity of the disease, the age of the patient and other factors, but generally ranges from 0.001 mg to 1000 mg, preferably 0.01 mg to 100 mg per kg body weight per dose as a total dose when the human TF inhibitor is a humanized anti-human TF antibody, for example.

The present application claims priority from Japanese Patent Application No. 2002-149494, the whole disclosure of which is incorporated by reference herein.

The following examples further illustrate the present invention without, however, limiting the scope of the invention thereto. Various changes and modifications can be made by those skilled in the art on the basis of the description of the invention, and such changes and modifications are also included in the present invention.

### EXAMPLES

### Example 1: Effects of blood coagulation factor complex products on the PT-prolonging action induced by a humanized anti-human TF antibody

### 1. Test materials

1) Freeze-dried human blood coagulation factor IX complexes
   (1)Proplex ST®
      Supplier: Baxter Healthcare Corporation
   (2)PPSB-HT "Nichiyaku"®
      Supplier: Nihon Pharmaceutical Co., Ltd.
2) Activated prothrombin complex concentrate product
   Autoplex®
   Supplier: Baxter Healthcare Corporation
3) Freeze-dried concentrate human blood coagulation factor IX
   Novact M®
   Supplier: The Chemo-Sero-Therapeutic Research Institute (Kaketsuken)
4) Recombinant blood coagulation factor VIII product
   Recombinate®
   Supplier: Baxter Healthcare Corporation
5) Heated human plasma protein product
   KENKETU ALBUMINATE-NICHIYAKU®
   Supplier: Nihon Pharmaceutical Co., Ltd.

### 2. Humanized anti-human TF antibody

A humanized anti-human TF antibody prepared by the method described in WO99/51743 and comprising the humanized H chain version shown in SEQ ID NO: 1 and the humanized L chain version shown in SEQ ID NO: 2 was used (supplied from Chugai Pharmaceutical). The antibody is known to recognize the complex of human TF and blood coagulation factor VII in addition to human TF, as described in Example 5 in the specification of WO99/51743. The humanized anti-human TF antibody was stored in a diluent (20 mmol/L sodium acetate, 150 mmol/L NaCl, pH 6.0).

### 3. Sample preparation protocol

1) The humanized anti-human TF antibody was prepared by diluting the humanized anti-human TF antibody stock in the diluent to a concentration of 1 mg/mL, 2 mg/mL or 3.33 mg/mL.
2) Proplex ST® was prepared at a concentration of 50 U/mL in the accompanying water for injection.
3) PPSB-HT® was prepared at a concentration of 50 U/mL in the accompanying water for injection.
4) Autoplex® was prepared at a concentration of 50 U/mL in the accompanying water for injection.
5) Novact M® was prepared at a concentration of 100 U/mL or 50 U/mL in the accompanying water for injection (100 U/mL was used in combination experiments with Recombinate).
6) Recombinate® was prepared at a concentration of 100 U/mL or 50 U/mL in the accompanying water for injection (100 U/mL was used in combination experiments with Novact M).
7) KENKETU ALBUMINATE-NICHIYAKU® was prepared at a concentration of 44 mg/mL as human serum albumin without using a diluent.

Albumate was included in test materials in view of the potential presence of a blood coagulation factor because it was formulated from human plasma though it is not a blood coagulation factor complex product used in the neutralizers or suppressants of the present invention.

### 4. Test method

1) In a syringe for blood collection containing a 3.8 w/v % sodium citrate solution (a 10 w/v % aqueous sodium citrate solution diluted in water for injection), blood was collected from healthy volunteers in an amount 9 times that of the solution.
2) The collected blood was transferred to a 50 mL centrifuge tube, where it was centrifuged at 2000 x g, 4°C for 10 minutes.
3) After centrifugation, the supernatant was transferred to another 50 mL centrifuge tube and used as citrate plasma.
4) The citrate plasma, humanized anti-human TF antibody, humanized anti-human TF antibody diluent and 6 types of blood products were mixed as shown in Table 1 below to prepare test samples in a total volume of 500 µL.

**Table 1-1**

| Samples without blood products | | | | |
|---|---|---|---|---|
| Citrate plasma (µL) | Humanized anti-human TF antibody | | Antibody diluent (µL) | Antibody concentration (µg/mL) |
| | Concentration (mg/mL) | Volume (µL) | | |
| 497.2 | | | 2.82 | |
| 497.2 | 1 | 2.82 | | 5.64 |
| 497.2 | 2 | 2.82 | | 11.3 |
| 497.2 | 3.33 | 2.82 | | 18.8 |

**Table 1-2**

| Samples containing a blood product Proplex ST, PPSB-HT, Autoplex, Novact M, or Recombinate | | | | | | | |
|---|---|---|---|---|---|---|---|
| Citrate plasma (µL) | Humanized anti-human TF antibody | | Antibody diluent (µL) | Blood product | | Antibody conc. (µg/mL) | Concentration of blood coagulation factor VIII or IX (U/mL) |
| | conc. (mg/mL) | vol. (µL) | | conc. (U/mL) | vol. (µL) | | |
| 478.4 | | | 2.82 | 50 | 18.8 | | 1.88 |
| 478.4 | 1 | 2.82 | | 50 | 18.8 | 5.64 | 1.88 |
| 478.4 | 2 | 2.82 | | 50 | 18.8 | 11.3 | 1.88 |
| 478.4 | 3.33 | 2.82 | | 50 | 18.8 | 18.8 | 1.88 |

**Table 1-3**

| Samples containing a combination of blood products Novact M and Recombinate | | | | | | | | | | |
|---|---|---|---|---|---|---|---|---|---|---|
| Citrate plasma (µL) | Humanized anti-human TF antibody | | Humanized anti-humanTF antibody | Novact M | | Recombinate | | Antibody conc. (µg/mL) | Blood coagulation factor VIII conc. (U/mL) | Blood coagulation factor IX conc.(U/mL) |
| | conc. (mg/mL) | vol. (µL) | | conc. (U/mL) | vol. (µL) | conc. (U/mL) | vol. (µL) | | | |
| 478.4 | | | 2.82 | 100 | 9.4 | 100 | 9.4 | | 1.88 | 1.88 |
| 478.4 | 1 | 2.82 | | 100 | 9.4 | 100 | 9.4 | 5.64 | 1.88 | 1.88 |
| 478.4 | 2 | 2.82 | | 100 | 9.4 | 100 | 9.4 | 11.3 | 1.88 | 1.88 |
| 478.4 | 3.33 | 2.82 | | 100 | 9.4 | 100 | 9.4 | 18.8 | 1.88 | 1.88 |

**Table 1-4**

| Samples containing Albuminate | | | | | | |
|---|---|---|---|---|---|---|
| Citrate plasma (µL) | Humanized anti-human TF antibody | | Antibody diluent (µL) | Albuminate (µL) | Antibody conc. (µg/mL) | Albuminate (vol %) |
| | conc. (mg/mL) | vol. (µL) | | | | |
| 419.6 | | | 2.82 | 77.6 | | 15.6 |
| 419.6 | 1 | 2.82 | | 77.6 | 5.64 | 15.6 |
| 419.6 | 2 | 2.82 | | 77.6 | 11.3 | 15.6 |
| 419.6 | 3.33 | 2.82 | | 77.6 | 18.8 | 15.6 |

5) The concentrations of the humanized anti-human TF antibody were calculated as follows. Given that the specific gravity of blood is 1, blood makes up 1/13 of the body weight and that Ht (hematocrit: the ratio of the volume of red blood cells in blood) is 42 vol %, the volume of plasma per kg body weight is 44.6 mL, which is equivalent to 53.2 mL of citrate plasma. When the humanized anti-human TF antibody is administered i.v. at doses of 0.3, 0.6, and 1.0 mg/kg, therefore, the concentrations of the humanized anti-human TF antibody in citrate plasma are 0.3 x 1000/53.2 = 5.64, 0.6 x 1000/53.2 = 11.3, and 1.0 x 1000/53.2 = 18.8 µg/mL, respectively.
6) The concentrations of the blood products were calculated as follows. Similarly to 5), the concentration of blood coagulation factor IX in citrate plasma is 100/53.2 = 1.88 U/mL when Proplex ST (as blood coagulation factor IX) is administered i.v. at the clinical maximum dose of 100 U/kg. The concentrations of PPSB-HT (as blood coagulation factor IX), Autoplex (as corrected blood coagulation factor VIII inhibitor activity), Novact M (blood coagulation factor IX) and Recombinate (blood coagulation factor VIII) were also calculated in the same manner. When Albuminate (as human serum albumin) is administered i.v. at the clinical maximum dose of 8.3 mL/kg (= 500 mL/60 kg i.v.), the concentration of albumin in citrate plasma is 8.3 x 100/53.2 = 15.6 vol %.
7) The prothrombin time (PT) of the test samples was measured by a fully automated blood coagulation analyzer AMAX (AMELUNG) once for each sample. However, the PT of drug-free citrate plasma was measured once at each of the beginning and the end of the PT test using each human citrate plasma sample, and the first PT value was used for the calculation of the average and standard error while the last PT value was used for confirmation.
8) The PT test by AMAX was performed as follows. In a test tube preliminarily warmed at 37°C was added 0.05 mL of citrate plasma and warmed at 37°C for 1 minute and then, 0.1 mL of Thromborel S reagent (human placental thromboplastin: from Dade Behring) warmed at 37°C was added to measure the time required for the reaction solution to clot (PT).

### 5. Method for statistical analyses

The effects of the blood products on PT in the presence of the humanized anti-human TF antibody at concentrations of 0.00, 5.64, 11.3 and 18.8 µg/mL were analyzed by a paired t test, and the dose-dependent effect of the humanized anti-human TF antibody on PT was analyzed by the Jonckheere test. The paired t test was calculated using Microsoft Excel 7.0 and the Jonckheere test for dose dependency was calculated using SAS.

### 6. Results

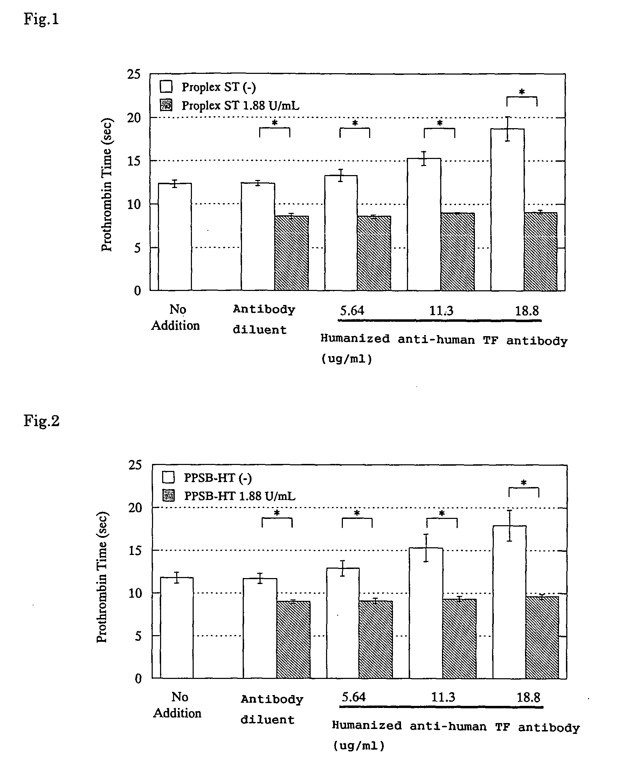

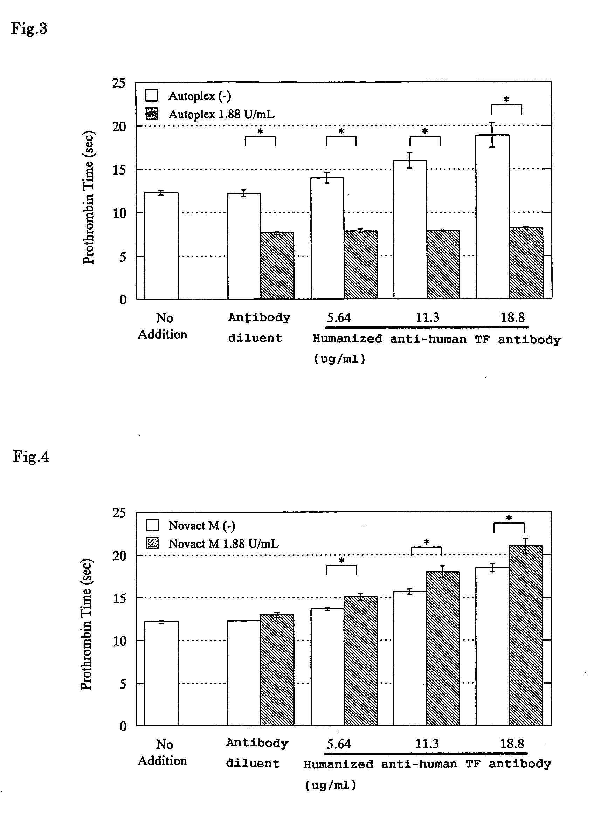

The results obtained are shown in Figures 1 to 7. Each figure shows the PT of human citrate plasma treated with the test material (striped bars) or not (blank bars). Data are shown as the mean ± standard error (S.E.) (n=3). * means P<0.05 as compared with the sample in which the test material is not contained.

In the presence of 1.88 U/mL (equivalent to 100 U/kg administered i.v.) of Proplex ST as a therapeutic agent for hemophilia B inhibitors and hemophilia A inhibitors, PPSB-HT as a therapeutic agent for hemophilia B inhibitors, or Autoplex as a therapeutic agent for hemophilia A inhibitors, the PT-prolonging action induced by the humanized anti-human TF antibody disappeared and PT decreased to levels below the normal range (11-13 seconds) (Figures 1 to 3).

The effects of products containing a single blood coagulation factor such as Novact M (blood coagulation factor IX product) and Recombinate (blood coagulation factor VIII product) and a combination of these two products on the PT-prolonging action induced by the humanized anti-human TF antibody were also evaluated in the same manner.

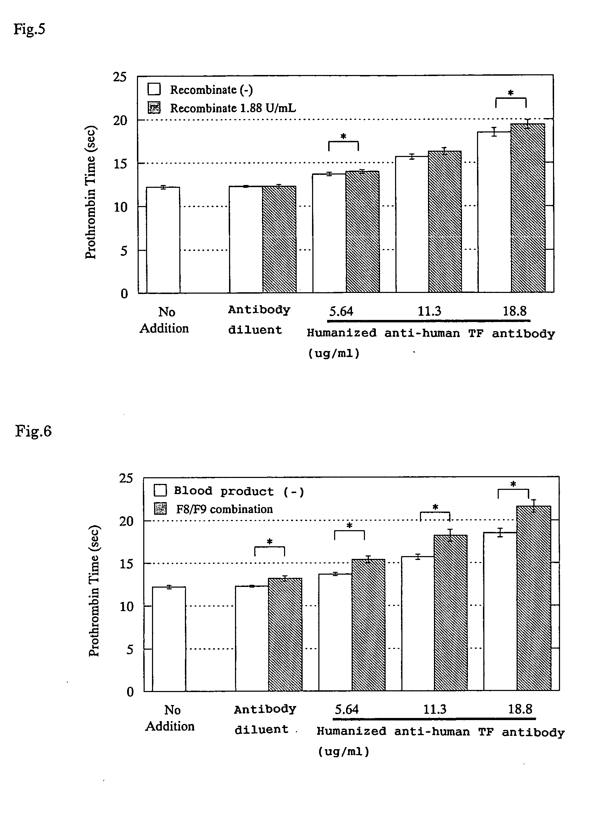

In the presence of 1.88 U/mL (equivalent to 100 U/kg administered i.v.) of Novact M or Recombinate, the PT-prolonging action induced by the humanized anti-human TF antibody was not suppressed (Figures 4, 5). When Novact M and Recombinate were used in combination, the PT-prolonging action induced by the humanized anti-human TF antibody was not suppressed (Figure 6). In the presence of these two products alone or in combination, the PT-prolonging action induced by the humanized anti-human TF antibody was found to rather significantly increase. In addition, PT was also significantly prolonged when these two products were used in combination even in the absence of the humanized anti-human TF antibody. These results suggest that at least blood coagulation factors VIII and IX play a minor role in the disappearance of the PT-prolonging action induced by the humanized anti-human TF antibodies in blood products.

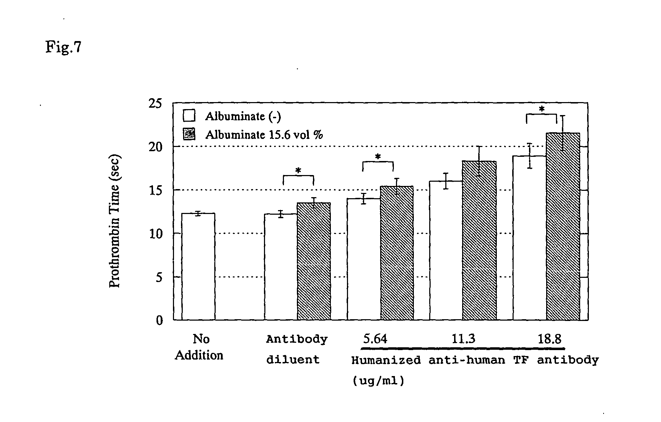

In the presence of 15.6 vol % (equivalent to 8.3 mL/kg administered i.v.) of Albuminate, it was found that the PT-prolonging action induced by the humanized anti-human TF antibody was not suppressed but rather significantly increased (except for the effect in the presence of 11.3 µg/mL of the humanized anti-human TF antibody) (Figure 7). In addition, PT was significantly prolonged even in the absence of the humanized anti-human TF antibody. This result showed that Albuminate had no coagulation effect that suppresses the PT-prolonging action induced by the humanized anti-human TF antibody.

It can be concluded from these results that Proplex ST, PPSB-HT and Autoplex neutralize the PT-prolonging action induced by the humanized anti-human TF antibody whereas Novact M, Recombinate, a combination of both and Albuminate do not neutralize the PT-prolonging action induced by the humanized anti-human TF antibody. Thus, it was found that the use of blood coagulation factor complexes Proplex ST, PPSB-HT and Autoplex is effective for improving bleeding tendency if it is observed by the administration of the humanized anti-human TF antibody.

### Example 2: Influence of an activated blood coagulation factor VII product on the PT-prolonging action induced by a humanized anti-human TF antibody

### 1. Materials

- 3.8 w/v % Sodium citrate: Citramin "FUSO" (Fuso Pharmaceutical Industries, Ltd.)
- Human citrate plasma: from healthy volunteers. A mixed solution of 2.0 mL of 3.8 w/v % sodium citrate and 18.0 mL of blood was centrifuged at 2000 x g (4°C) for 10 minutes, and the supernatant, i.e. citrate plasma ([1]) was collected. It was allowed to stand on ice.
- Recombinant activated blood coagulation factor VII product: 1.2 mg of "NovoSeven" for injection (Novo Nordisk). On the day of the experiments, it was dissolved in the accompanying diluent following the package leaflet (0.6 mg/mL = 30 KIU/mL activated blood coagulation factor VII (F.VIIa): solution [2]).
   Given that NovoSeven becomes totally distributed in blood when it is administered at the maximum single dose of 120 µg/kg = 6 KIU/kg indicated in the package leaflet, the concentration of NovoSeven in citrate plasma is 6 KIU/kg (53.2 mL/kg = 0.113 KIU/mL). As described below, the present experiments were performed by adding NovoSeven to human citrate plasma to an F.VIIa concentration of 0.113 KIU/mL.
- PT reagent: Thromborel S (Dade Behring). In accordance with the instructions in the package leaflet, it was dissolved in 4.0 mL/bottle of Milli-Q water and allowed to stand in a CO₂ incubator (CDP1700 series, Hirasawa) at 37°C for 15 minutes. Then, it was placed at a given position in a fully automated coagulation analyzer.
- Humanized anti-human TF antibody solutions: A diluent was used to prepare a 5000 µg/mL humanized anti-human TF antibody solution (solution [3]), a 1500 µg/mL humanized anti-human TF antibody solution (solution [4]), and a 500 µg/mL humanized anti-human TF antibody solution (solution [5]).
   The diluent used was 20 mmol/L sodium acetate, 150 mmol/L NaCl [pH 6.0] ([6]).

### 2. Test method

(1) Test samples were prepared as follows.
   - 0 µg/mL humanized anti-human TF antibody sample [7]:
      990 µL [1] + 10 µL [6]
   - 5.0 µg/mL humanized anti-human TF antibody sample [8] (equivalent to 0.266 mg/kg immediately after i.v. bolus administration):
      990 µL [1] + 10 µL [5]
   - 15 µg/mL humanized anti-human TF antibody sample [9] (equivalent to 0.798 mg/kg immediately after i.v. bolus administration):
      990 µL [1] + 10 µL [4]
   - 50 µg/mL humanized anti-human TF antibody sample [10] (equivalent to 2.66 mg/kg immediately after i.v. bolus administration):
      990 µL [1] + 10 µL [3]
   - 0 µg/mL humanized anti-human TF antibody / 0.113 KIU/mL F.VIIa sample:
      264.5 µL [7] + 1.0 µL [2]
   - 5.0 µg/mL humanized anti-human TF antibody / 0.113 KIU/mL F.VIIa sample:
      264.5 µL [8] + 1.0 µL [2]
   - 15 µg/mL humanized anti-human TF antibody / 0.113 KIU/mL F.VIIa sample:
      264.5 µL [9] + 1.0 µL [2]
   - 50 µg/mL humanized anti-human TF antibody / 0.113 KIU/mL F.VIIa:
      264.5 µL [10] + 1.0 µL [2]
(2) The prothrombin time (PT) was measured by the fully automated coagulation analyzer.

### 3. Results

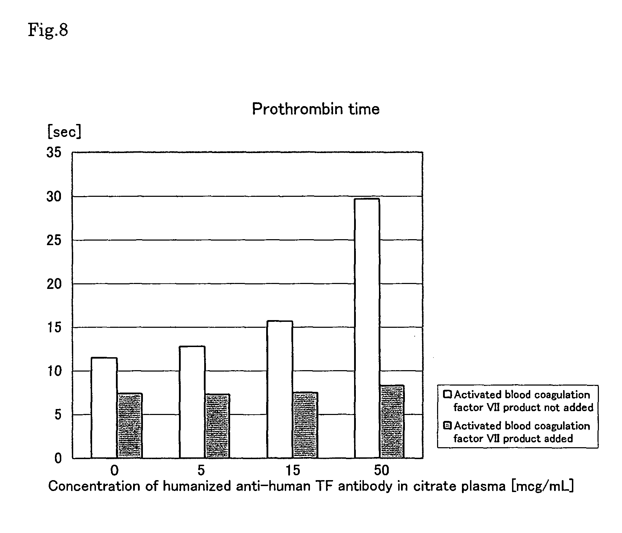

The results are shown in Figure 8. When F.VIIa was not added, the blood coagulation time (prothrombin time) was found to be prolonged dependently on the dose of the humanized anti-human TF antibody. This shows the anticoagulation activity of the humanized anti-human TF antibody.

When F.VIIa was added at a clinical dose (the maximum value given in the package leaflet), the PT exceeded the normal range (11-13 seconds) and therefore, the blood coagulation time prolonging action induced by the humanized anti-human TF antibody was completely neutralized.

Thus, the anticoagulation activity of the humanized anti-human TF antibody was shown to be neutralized by F.VIIa.

### Example 3: Influence of an humanized anti-human TF antibody on the PT-shortening action induced by an activated blood coagulation factor VII product

### 1. Materials

- 3.8 w/v % Sodium citrate: Citramin "FUSO" (Fuso Pharmaceutical Industries, Ltd.)
- Human citrate plasma: from healthy volunteers. A mixed solution of 2.0 mL of 3.8 w/v % sodium citrate and 18.0 mL of blood was centrifuged at 2000 x g (4°C) for 10 minutes, and the supernatant, i.e. citrate plasma ([1]) was collected. It was allowed to stand on ice.
- Recombinant activated blood coagulation factor VII product: 1.2 mg of "NovoSeven" for injection (Novo Nordisk). On the day of the experiments, it was dissolved in the accompanying diluent in accordance with the instructions in the package leaflet (0.6 mg/mL = 30 KIU/mL activated blood coagulation factor VII (F.VIIa): solution [2]).
   Given that NovoSeven becomes totally distributed in blood when it is administered at the maximum single dose of 120 µg/kg = 6 KIU/kg indicated in the package leaflet, the concentration of NovoSeven in citrate plasma is 6 KIU/kg (53.2 mL/kg = 0.113 KIU/mL). As described below, the present experiments were performed by adding NovoSeven to human citrate plasma to an F.VIIa concentration of 0.113 KIU/mL.
- PT reagent: Thromborel S (Dade Behring). In accordance with the instructions in the package leaflet, it was dissolved in 4.0 mL/bottle of Milli-Q water and allowed to stand in a CO₂ incubator (CDP1700 series, Hirasawa) at 37°C for 15 minutes. Then, it was placed at a given position in a fully automated coagulation analyzer.
- APTT reagent: Activated Cephaloplastin Reagent (Dade Behring).
- Humanized anti-human TF antibody solution: 15.4 mg/mL [3] (diluent: 20 mmol/L sodium acetate, 150 mmol/L NaCl [pH 6.0]).

### 2. Test method

(1) Test samples were prepared as follows.
   - 0.113 KIU/mL F.VIIa sample [4]:
      1054 µL [1] + 4.0 µL [2]
   - 0.113 KIU/mL F.VIIa / 100 µg/mL humanized anti-human TF antibody sample (equivalent to 5.32 mg/kg antibody immediately after i.v. bolus administration):
      198.7 µL [4] + 1.3 µL [3]
   - 0.113 KIU/mL F.VIIa / 200 µg/mL humanized anti-human TF antibody sample (equivalent to 10.64 mg/kg antibody immediately after i.v. bolus administration):
      197.4 µL [4] + 2.6 µL [3]
   - 0.113 KIU/mL F.VIIa / 400 µg/mL humanized anti-human TF antibody sample (equivalent to 21.28 mg/kg antibody immediately after i.v. bolus administration):
      194.8 µL [4] + 5.2 µL [3]
(2) The prothrombin time (PT) was measured by the fully automated coagulation analyzer.

### 3. Results

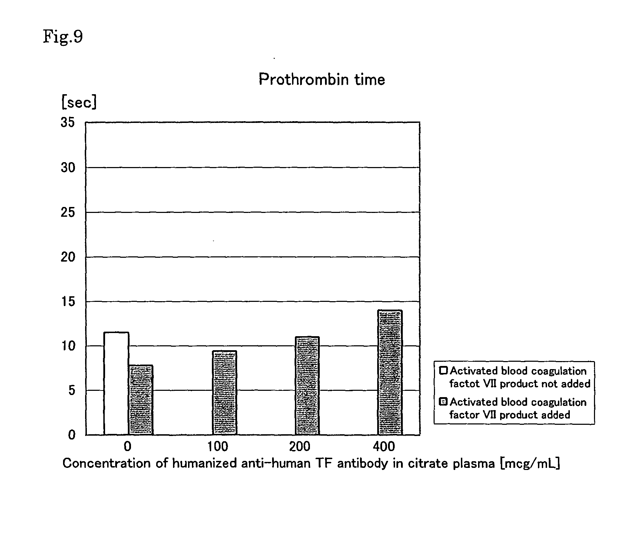

The results are shown in Figure 9. A clinical dose (the maximum value given in the package leaflet) of F.VIIa shortened the blood coagulation time (prothrombin time). This shows the blood coagulation-promoting action of F.VIIa.

When the humanized anti-human TF antibody was added here, the blood coagulation-promoting action of F.VIIa was dose-dependently suppressed. Moreover, it was recovered to the normal range (11-13 seconds) in the presence of 200 µg/mL of the humanized anti-human TF antibody.

Thus, the blood coagulation-promoting action of F.VIIa was shown to be neutralized by the humanized anti-human TF antibody.

### Example 4: Influence of a humanized anti-human TF antibody and low molecular weight heparin on rebleeding from surgical wounds

Macaques (Chinese origin, 7 males / 1 female, age 3-6, body weight: 2.51-5.98 kg) were anesthetized by intramuscular administration of Ketalar (0.7 mL, 35 mg as ketamine). Each animal was weighed and then fixed spine on an operating table. An inhalation mask was put on to cover the nostrils and mouth, and inhalation anesthesia was performed with 1 vol % of a mixed isoflurane / dinitrogen monoxide gas (2 volumes of dinitrogen monoxide : 1 volume of oxygen). The neck, femurs and legs were shaved.

An indwelling needle was placed in each of the right and left lateral saphenous veins and physiological saline was administered by infusion at 1.0 mL/kg/h (for administration and for blood collection) until the end of the experiment. A thermometer was placed into the rectum from the anus, and the rectal temperature was maintained at or higher than 35.0°C by inserting a heating pad locally under the individual, if necessary. The skin of either one of the right or left femur was incised and muscle layers were retracted to expose the femoral artery. A catheter was inserted into the femoral artery to measure the arterial blood pressure.

The center of the neck was incised along the midline and muscle layers were retracted to expose the left common carotid artery and left internal jugular vein. Minor bleeding was treated by applying a pressure dressing (hemostasis by direct pressure with gauze). If necessary, an electric coagulator was used to stop bleeding, but its use was limited to a minimum. After the completion of the incision, the incision site in the neck was checked to confirm that bleeding had completely stopped.

After blood in the incision site in the neck was completely wiped, the animal was allowed to rest for 1 hour, during which rebleeding blood from the incision site in the neck was collected with gauze paper.

Then, 0.3 mg/kg of a humanized anti-human TF antibody, or 75 IU/kg of low molecular weight heparin (Fragmin, Pharmacia Upjohn), and/or the diluent for each of them (20 mmol/L sodium acetate, 150 mmol/L NaCl [pH 6.0] or physiological saline) was intravenously administered via an indwelling intravenous needle for administration.

After blood in the incision site in the neck was completely wiped, the animal was allowed to rest for 1 hour, during which rebleeding blood from the incision site in the neck was collected with gauze paper.

This procedure was repeated further three times with additional injection of the humanized anti-human TF antibody or low molecular weight heparin (at the doses indicated in the experimental results).

At the beginning of resting for 1 hour, blood was collected via an indwelling intravenous needle for blood collection.

The bleeding rate (mL/h) was calculated at each dose from the amount of hemoglobin in the collected blood and the hemoglobin concentration in the bloodstream measured with a Hemoglobin B test Wako.

The experimental results are as follows.

All individuals showed slight amounts of rebleeding per hour during the unmedicated period with no visible difference observed between groups. Specifically, the amounts of rebleeding were an average of 0.05 mL (with a standard error of ±0.01 mL) in the group treated with the humanized anti-human TF antibody (n=3), an average of 0.04 mL (with a standard error of ±0.01 mL) in the group treated with low molecular weight heparin (n=3), and an average of 0.04 mL in the vehicle group (n=2).

When the humanized anti-human TF antibody was administered to the animals in the group treated with the humanized anti-human TF antibody at doses of 0.3, 1.2, 6.0 and 30.0 mg/kg successively (cumulative totals of 0.3, 1.5, 7.5, 37.5 mg/kg), the amounts of rebleeding per hour were an average of 0.04 mL/h (with a standard error of ±0.00 mL/h), an average of 0.06 mL/h (with a standard error of ±0.01 mL/h), an average of 0.06 mL/h (with a standard error of ±0.02 mL/h), and an average of 0.08 mL/h (with a standard error of ±0.02 mL/h), respectively. Bleeding was observed as slight as was observed during the unmedicated period. Two-way ANOVA detected neither difference between individuals nor difference between doses in the rebleeding rate in the group treated with the humanized anti-human TF antibody (with p values of 0.3237 and 0.4676, respectively).

When low molecular weight heparin was administered to the animals in the group treated with low molecular weight heparin at doses of 75, 300, 1500 and 7500 IU/kg successively (cumulative totals of 75, 375, 1875 and 9375 IU/kg), however, the amounts of rebleeding per hour were an average of 0.17 mL/h (with a standard error of ±0.09 mL/h), an average of 1.52 mL/h (with a standard error of ±0.82 mL/h), an average of 2.91 mL/h (with a standard error of ±1.46 mL/h), and an average of 9.14 mL/h (with a standard error of ±4.55 mL/h), respectively. Two-way ANOVA detected no difference between individuals but differences between doses in the rebleeding rate in the group treated with low molecular weight heparin (with p values of 0.1393 and 0.0474, respectively). Thus, a paired trend test was performed on doses to show the dose dependency of low molecular weight heparin on the amount of rebleeding (p=0.0126).

When the diluent was administered to the vehicle group according to the same schedule as described above, the amounts of rebleeding per hour were an average of 0.06 mL/h, an average of 0.06 mL/h, an average of 0.07 mL/h, and an average of 0.08 mL/h, successively. Bleeding was observed as slight as during the unmedicated period. No statistical analysis was performed because the number of samples was 2.

These results showed that rebleeding does not occur even if the humanized anti-human TF antibody is administered up to 37.5 mg/kg (equivalent to 705 µg/mL as the concentration in citrate plasma) after hemostasis has been achieved once in surgical wounds.

## Claims

1. A formulation for neutralizing the anticoagulant action of a human tissue factor inhibitor, comprising a blood coagulation factor complex product or an activated blood coagulation factor VII product as an active ingredient.

2. The formulation of claim 1 wherein the human tissue factor inhibitor is an anti-human tissue factor antibody.

3. The formulation of claim 2 wherein the anti-human tissue factor antibody is a humanized anti-human tissue factor antibody.

4. The formulation of claim 3 wherein the humanized anti-human tissue factor antibody is a humanized anti-human tissue factor antibody comprising the humanized H chain version shown in SEQ ID NO: 1 and the humanized L chain version shown in SEQ ID NO: 2.

5. A formulation for suppressing the bleeding or bleeding tendency caused by a human tissue factor inhibitor, comprising a blood coagulation factor complex product or an activated blood coagulation factor VII product as an active ingredient.

6. A formulation for neutralizing the blood coagulation time (prothrombin time) prolonging action caused by a human tissue factor inhibitor, comprising a blood coagulation factor complex product or an activated blood coagulation factor VII product as an active ingredient.

7. A formulation for suppressing bleeding or bleeding tendency, comprising a blood coagulation factor complex product or an activated blood coagulation factor VII product as an active ingredient, **characterized in that** it is administered simultaneously with a human tissue factor inhibitor or after administering a human tissue factor inhibitor.

8. An antithrombotic agent comprising a human tissue factor inhibitor and a blood coagulation factor complex product or an activated blood coagulation factor VII product.

9. The antithrombotic agent of claim 8 wherein the human tissue factor inhibitor is an anti-human tissue factor antibody.

10. The antithrombotic agent of claim 9 wherein the anti-human tissue factor antibody is a humanized anti-human tissue factor antibody.

11. The antithrombotic agent of claim 10 wherein the humanized anti-human tissue factor antibody is a humanized anti-human tissue factor antibody comprising the humanized H chain version shown in SEQ ID NO: 1 and the humanized L chain version shown in SEQ ID NO: 2.

12. A pharmaceutical kit comprising a human tissue factor inhibitor and a blood coagulation factor complex product or an activated blood coagulation factor VII product.

13. The kit of claim 12 wherein the human tissue factor inhibitor is an anti-human tissue factor antibody.

14. The kit of claim 13 wherein the anti-human tissue factor antibody is a humanized anti-human tissue factor antibody.

15. The kit of claim 14 wherein the humanized anti-human tissue factor antibody is a humanized anti-human tissue factor antibody comprising the humanized H chain version shown in SEQ ID NO: 1 and the humanized L chain version shown in SEQ ID NO: 2.

16. A formulation for suppressing the blood coagulation-promoting action of an activated blood coagulation factor VII product, comprising a human tissue factor inhibitor as an active ingredient.

17. The formulation of claim 16 wherein the human tissue factor inhibitor is an anti-human tissue factor antibody.

18. The formulation of claim 17 wherein the anti-human tissue factor antibody is a humanized anti-human tissue factor antibody.

19. The formulation of claim 18 wherein the humanized anti-human tissue factor antibody is a humanized anti-human tissue factor antibody comprising the humanized H chain version shown in SEQ ID NO: 1 and the humanized L chain version shown in SEQ ID NO: 2.

20. A formulation for suppressing the thrombosis or hypercoagulable states caused by an activated blood coagulation factor VII product, comprising a human tissue factor inhibitor as an active ingredient.

21. A formulation for neutralizing the blood coagulation time (prothrombin time) shortening action caused by an activated blood coagulation factor VII product, comprising a human tissue factor inhibitor as an active ingredient.

22. A formulation for suppressing thrombosis or hypercoagulable states, comprising a human tissue factor inhibitor as an active ingredient, **characterized in that** it is administered simultaneously with an activated blood coagulation factor VII product or after administering an activated blood coagulation factor VII product.

23. A hemostatic agent comprising an activated blood coagulation factor VII product and a human tissue factor inhibitor.

24. The hemostatic agent of claim 23 wherein the human tissue factor inhibitor is an anti-human tissue factor antibody.

25. The hemostatic agent of claim 24 wherein the anti-human tissue factor antibody is a humanized anti-human tissue factor antibody.

26. The hemostatic agent of claim 25 wherein the humanized anti-human tissue factor antibody is a humanized anti-human tissue factor antibody comprising the humanized H chain version shown in SEQ ID NO: 1 and the humanized L chain version shown in SEQ ID NO: 2.

27. A method for neutralizing the anticoagulant action of a human tissue factor inhibitor, comprising administering a blood coagulation factor complex product or an activated blood coagulation factor VII product.

28. A method for suppressing the bleeding or bleeding tendency caused by a human tissue factor inhibitor, comprising administering a blood coagulation factor complex product or an activated blood coagulation factor VII product.

29. A method for neutralizing the blood coagulation time (prothrombin time) prolonging action caused by a human tissue factor inhibitor, comprising administering a blood coagulation factor complex product or an activated blood coagulation factor VII product.

30. A method for suppressing bleeding or bleeding tendency, comprising administering a blood coagulation factor complex product or an activated blood coagulation factor VII product, **characterized in that** it is administered simultaneously with a human tissue factor inhibitor or after administering a human tissue factor inhibitor.

31. A method for treating thrombosis, comprising administering a human tissue factor inhibitor and a blood coagulation factor complex product or an activated blood coagulation factor VII product.

32. A method for suppressing the blood coagulation-promoting action of an activated blood coagulation factor VII product, comprising administering a human tissue factor inhibitor.

33. A method for suppressing the thrombosis or hypercoagulable states caused by an activated blood coagulation factor VII product, comprising administering a human tissue factor inhibitor.

34. A method for neutralizing the blood coagulation time (prothrombin time) shortening action caused by an activated blood coagulation factor VII product, comprising administering a human tissue factor inhibitor.

35. A method for suppressing thrombosis or hypercoagulable states, comprising administering a human tissue factor inhibitor, **characterized in that** it is administered simultaneously with an activated blood coagulation factor VII product or after administering an activated blood coagulation factor VII product.

36. A method for stopping bleeding, comprising administering an activated blood coagulation factor VII product and a human tissue factor inhibitor.
